Europäisches Patentamt

European Patent Office

Office européen des brevets

(19)

(11) **EP 0 463 114 B1**

(12) **EUROPEAN PATENT SPECIFICATION**

(45) Date of publication and mention
of the grant of the patent:
**15.05.1996 Bulletin 1996/20**

(21) Application number: **90906028.7**

(22) Date of filing: **05.03.1990**

(51) Int Cl.6: **G01N 33/50**, G01N 33/564,
A61K 47/48

(86) International application number:
**PCT/US90/01197**

(87) International publication number:
**WO 90/10871 (20.09.1990 Gazette 1990/22)**

(54) **ASSAY FOR AND TREATMENT OF AUTOIMMUNE DISEASES**

TESTVERFAHREN FÜR UND BEHANDLUNG VON AUTOIMMUNKRANKHEITEN

ANALYSE DE DETECTION ET TRAITEMENT DE MALADIES AUTO-IMMUNES

(84) Designated Contracting States:
**AT BE CH DE DK ES FR GB IT LI LU NL SE**

(30) Priority: **06.03.1989 US 319499**

(43) Date of publication of application:
**02.01.1992 Bulletin 1992/01**

(73) Proprietor: **THE REGENTS OF THE UNIVERSITY
OF CALIFORNIA
Berkeley, California 94720 (US)**

(72) Inventors:
 • **CAHALAN, Michael, Daniel
  Laguna Beach, CA 92651 (US)**
 • **CHANDY, Kanianthara, George
  Irvine, CA 92715 (US)**
 • **GRISSMER, Stephen
  Irvine, CA 92715 (US)**

(74) Representative: **Orès, Bernard et al
Cabinet ORES
6, Avenue de Messine
F-75008 Paris (FR)**

(56) References cited:
 **WO-A-89/06967          WO-A-89/06968**

 • **BIOLOGICAL ABSTRACTS, vol. 87, no. 4, 1989,
  Philadelphia, PA (US); M.A. TILL et al., p. AB-167,
  no. 34914/**
 • **BIOLOGICAL ABSTRACTS, vol. 85, no. 7, 1988,
  Philadelphia, PA (US); F.E. KATZ et al., p.
  AB-651, no. 69872/**
 • **SCIENCE, vol. 233, September 1986; K.G.
  CHANDY et al., abstract/**
 • **JOURNAL OF IMMUNOLOGY, vol. 134, no. 2,
  February 1985; D. WOFSY et al., abstract/**
 • **Dorland's Illustrated Medical Dictionary, 26th
  ed., page 760**

## Description

### Field of the Invention

The present invention relates generally to advances made in the field of autoimmune disease prognosis, diagnosis and treatment. More particularly, the present invention is directed to certain assays for, and consequent treatment of, autoimmune diseases based upon the identification and characterization herein of a unique ion channel aberration found to be associated with abnormal T cells linked to such diseases. Assays, prognostic markers and therapeutic modalities for such autoimmune disease states are defined and described.

### Background of the Invention

Autoimmune diseases have been the subject of widespread press attention because of the considerable morbidity worldwide that they cause. Autoimmune diseases include rheumatoid arthritis, type-1 diabetes mellitus (insulin dependent), multiple sclerosis, myasthenia gravis, systemic lupus erythematosus, Sjogren's syndrome, mixed connective tissue disease, experimental allergic encephalomyelitis (EAE), to name a few. Considerable research has been expended and is currently underway in order not only to devise a treatment or prophylaxis against such devastating diseases, but also to study the underlying etiology(ies) such that a better understanding can be gained as to common denominators, if any, that would more directly focus a plan of attack for conquering them.

In most cases, it is believed that autoimmune diseases result from abnormal cells of the immune system destroying 5 target tissues, either by direct killing or by producing autoantibodies. One may focus for exemplification on historic autoimmune diseases. One such is the so-called systemic lupus erythematosus (SLE). In SLE, abnormal B-lymphocytes produce anti-DNA antibodies that are positively charged and aggregate on negatively charged kidney cells causing inflammation and nephritis, which is symptomatic of SLE. In diabetes mellitus, abnormal T cells systematically destroy pancreatic islet cells such that they prove incapable of producing insulin, a necessary hormone for proper metabolic balance in an organism. In multiple sclerosis, abnormal T cells are believed to damage myelin basic protein, a major component of nerve cells, which systematically destroys certain nerve cells, causing a spectrum of neurological symptoms. In the autoimmune diseases studied to date, there seems to emerge a common pattern of abnormal immune system cells producing materials that either destroy or retard certain target tissues causing symptoms manifest for that disease state.

Current treatment for these diseases remains on an empirical level and is based on causing generalized immunosuppression, either with steroids or other immunosuppressive drugs. This therapeutic approach is also fraught with other problems including associated severe side effects. Further, they serve only to retard the natural progression of these autoimmune diseases. Effective therapeutic treatment, to say nothing of a cure, is beyond present day medical technology. The aberrations in the immune system resulting in these various autoimmune diseases are not well understood, despite the extensive research that has taken place in this field. See Theofilopoulos, et al., Adv. Immunol. 37, 269 (1985), for example.

Research has focused on the use of various murine models that have provided considerable insight into the pathogenesis of the disease states, although the clinical syndromes and immunological abnormalities vary considerably from strain to strain, making them less than perfect studies. Thus, a common underlying cellular or molecular defect that is common to all these diseases has not been identified, if indeed there is even a suggestion in the extant art that one exists.

Studies by several investigators have demonstrated that injection of antibodies against CD4$^+$CD8$^-$ T cells prevents, and in some cases retards, the onset of certain autoimmune diseases in certain murine models. See Theofilopoulos et al., Adv. Immunol. 37, 269 (1985); Wofsy et al., J. Exp. Med. 161, 378 (1985); Wofsy et al., J. Immunol. 136, 4554 (1986); Wofsy et al., J. Immunol. 134 852 (1985); Santoro et al., J. Exp. Med. 167, 1713 (1988); Waldor et al., Science 227, 415 (1985); Ranges et al., J. Exp. Med. 162, 1105 (1985); Christadoss et al., J. Immunol. 136, 2437 (1986); and Sriram et al., J. Immunol. 136, 4464 (1986). This treatment also results in the reduction of a subpopulation of CD4$^-$CD8$^-$ T cells. In cumulative effect, these data may point to a common underlying mechanism mediated by CD4$^+$CD8$^-$ T cells and/or CD4$^-$CD8$^-$ T cells. See Santoro, et al., J. Exp. Med. 167, 1713 (1988).

Existing evidence may further suggest reasons attending the role CD4$^-$CD8$^-$ T cells play in the induction of SLE, for example. First, these cells from diseased SLE mice induce B cells to secrete pathogenic anti-DNA autoantibodies in vitro, whereas the same cells from normal mice or from pre-autoimmune mice do not exhibit this property. Datta, et al., J. Exp. Med. 165, 1252 (1987); Sainis et al., J. Immunol. 140, 2215 (1988). Second, the onset of SLE is greatly accelerated in mice that have a severe lymphoproliferation of such cells due to the expression of the autosomal recessive mutations lpr or gld. See Theofilopoulos, et al., Supra. Third, patients with SLE nephritis have an expanded CD4$^-$CD8$^-$ T cells population in their blood that induces secretion of autoantibodies. Shivakumar et al., FASEB J. 3, A492 (No. 1548) (Feb., 1989) and Datta, Federation of American Societies for Experimental Biology Summer Conference on Autoimmunity at Saxton's River, Vermont, 3-8 July, 1988. Cumulatively, these data may suggest that CD4$^-$CD8$^-$ T cells mediate in some fashion autoimmune disease, contributing to the development of the disease. However, thus far, no marker or alteration or aberration that is unique to the abnormal CD4⁻CD8⁻ T cells related to autoimmunity, has been identified.

It was previously reported that K⁺ channel expression in proliferating CD4⁻CD8⁻ T cells from mice with lpr mutations was dramatically altered. Chandy, et al., Science 233, 1197 (1986). That research resulted in the association of an abnormal pattern of ion channel expression with an SLE prone genetic defect in cells of the immune system, associated with abnormal lymphoproliferation.

The next step was to determine whether the same abnormal expression pattern could be observed for cells from mice that were not genetically predisposed to SLE, as were the murine models of Chandy, et al., Supra. Thus, in Grissmer, et al., Journal of Immunology 141, 1137 (1988), murine models that were not prone for SLE, developed similar abnormal expression patterns associated with the onset of SLE and lymphoproliferation. Thus, while in Chandy, et al., Supra, abnormal overexpression of type I K⁺ channels in SLE prone murine models was thought to be a lpr mutation dependent abnormality of the immune system, in Grissmer, et al., Supra, similar proliferation of type 1 K+ channels in mice with two distinct mutations (lpr and gld) indicated either that such overexpression was somehow associated with SLE itself or to simple lymphoproliferation symptoms. See also Lewis, et al., Science 239, 771 (1988) and DeCoursey, et al., Nature 307, 465 (1984).

Theofilopoulos, et al., in Advances in Immunology 37, 269 (1985), a review article, suggest that the disease profile in the lpr and gld murine models does not resemble any known variant of human SLE. Theofilopoulos, et al. suggest that a clear correlative model is necessary.

It is thus a goal in the art to perform experiments with more representative models in order to establish whether, and if so how, abnormal immune cell proliferation and ion channel expression is related etiologically to autoimmune diseases.

Therefore, present attention focused on the ion channel itself. Three types of ion channel types, classified pharmacologically and electrophysiologically, were identified, the so-called n, n' and I types. T cells in the peripheral lymphoid tissues for present purposes, are characterized into relevant types: CD4⁺CD8⁻, CD4⁻CD8⁺, CD4⁻CD8⁻. The CD4⁺CD8⁻ cells are thought to express approximately 20 n channels per cell and are believed to have no n' and I channels. CD4⁻CD8⁺ T cells are thought to have about 20 n' and I channels per cell and no n channels. And CD4⁻CD8⁻ T cells are believed to express about 20 channels per cell, all three types being represented. In a normal immune response reflecting induction of activity, such as with mitogens, the n channel types are increased upwards of ten fold in the cells that are activated. Thus, normal T cells when stimulated by mitogens, show as a normal immune response elevation in the number of n channels. Blocking these n channels, for example with tetraethyl ammonium (TEA), would serve to shut down and effectively block an immune response. Abnormal T cells may also be subject to a similar blockage. However, such abnormal T cells are represented by double negative CD4⁻ CD8⁻ cells, manifesting a proliferation of type I channels that seem implicated in the induction of autoimmune disease states. Type I K⁺ channels are not use dependent, close more rapidly on repolarization than do n channels and are much more sensitive to blockage, for example by TEA. It was the goal of the present research to establish a link, if any, hence an etiology between abnormal ion channel expression in immune cells and systemic symptoms of autoimmune disease.

## Summary of the Invention

The threshold experiment establishing the aforementioned link was performed with murine models that are representative of human SLE, namely murine models NZBxNZW, NZBxSWR, BXSB-Yaa and MRL +/+. In these models, it was found that in the case of SLE, as one example of an autoimmune disease, the unique pattern of type I K⁺ channel - -expression in CD4⁻CD8⁻ T cells was associated with the onset of SLE, rather than it being a result of abnormal lymphoproliferation. Thus, the link was established eliminating the possibility that what was being observed in earlier studies was simply symptomatic of the lymphoproliferative autoimmune disease.

The present invention is predicated upon the identification and characterization of an etiology of autoimmune diseases, represented by SLE, type-1 diabetes mellitus, experimental allergic encephalomyelitis (EAE), and others, namely, that all such autoimmune diseases demonstrate the presence of abnormal CD4⁻CD8⁻ T cells expressing large numbers of type I K⁺ ion channels. Thus, such channel expression parallels the ability to induce autoimmunity. Having established such a link as a predicate of the present invention, it intellectually follows that the present invention is directed to several consequential aspects: 1) an assay for diagnosing autoimmune diseases by measuring the relative numbers of type I K⁺ channels in abnormal CD4⁻CD8⁻ T cells as defined in independent Claim 4, 2) an assay for testing extrinsic materials for their ability to selectively modulate type I K⁺ channels in abnormal CD4⁻CD8⁻ T cells as defined in independent Claim 1, 3) the use of a selected identified extrinsic material for the preparation of a drug useful for treating an autoimmune disease as defined in independent Claim 7, and 4) a process, as defined in independent Claim 16, for preparing a composition containing such selected identified extrinsic material and the use, as defined in independent Claim 17, of a composition obtained by the said process.

Thus, the present invention is predicated upon the identification and characterization of a diagnostic marker for autoimmune diseases, that leads to a diagnostic and a therapeutic target for such diseases. This predicate makes possible the identification of the etiology of a given autoimmune disease state, an assay to detect presence of the marker, an assay to identify (selective) modulators of the marker, suitable modulators identified from such an assay and therapeutic modalities based upon such modulators (drugs) and/or specific antibodies raised against type 1 $K^+$ channels of abnormal $CD4^-CD8^-$ T cells.

Thus, the quintessence of the present invention enables in turn the consequence that the identified elevated numbers of type $\underline{l}$ $K^+$ channels in abnormal $CD4^-CD8^-$ T cells can be exploited by a method of screening for extrinsic materials having a modulating effect on such channels comprising providing an in vitro system containing elevated type $\underline{l}$ $K^+$ channels of abnormal $CD4^-CD8^-$ T cells, challenging such cells with one or more of a battery of test materials having the putative potential to modulate said type $\underline{l}$ $K^+$ channels, monitoring the effect of said test materials on said $\underline{l}$ $K^+$ channels and selecting candidates capable of modulating type $\underline{l}$ $K^+$ channels.

The assay for determining presence of an autoimmune disease of the invention comprises providing T cells containing $K^+$ channels from a test individual, identifying abnormal T cells from among the population of the provided T cells, measuring the relative numbers of type $\underline{l}$ $K^+$ channels of the abnormal cells, and determining whether said type $\underline{l}$ $K^+$ channels are elevated or normal.

The drug prepared from an extrinsic material according to the invention can be used for treating autoimmune diseases that are symptomatized by elevated numbers of type $\underline{l}$ $K^+$ channels in abnormal $CD4^-CD8^-$ T cells of the immune system of the organism manifesting said autoimmune disease in contacting said organism and/or said immune cell system with the said drug having a modulating effect on said type $\underline{l}$ $K^+$ channels, such materials optionally identified via the assay system described Supra. In particular, autoimmune diseases as described above, selectively without disturbing type n and $\underline{n}'$ ion channels expression characteristic of normal immune response can be treated as described above with, as an example, tetraethyl ammonium (TEA) or such other selective modulator defined and optionally selected via the assay described supra.

The present invention is thus directed to the identification, management and control of autoimmune diseases, including:

(1) use as a prognostic tool, for example, by making reactive antibodies to the type $\underline{l}$ $K^+$ channels and measuring the numbers thereof, and/or
(2) selectively screening for, preferably selective, modulators of type $\underline{l}$ $K^+$ channels for use as a diagnostic, and/or
(3) blocking, retarding or eliminating selectively type $\underline{l}$ $K^+$ channels for use as a therapeutic.

In respect of the above, it is contemplated that one could link a reactive antibody with a cell toxin such that the antibody would locate and bind to a type $\underline{l}$ $K^+$ channel of an aberrant $CD4^-CD8^-$ T cell (responsible for an autoimmune disease) and thereby place the cell toxin, e. g., ricin, in close proximity to the target abnormal cell with consequential cytotoxic activity. One could use Psoralen to image the aberrant target cells followed by activation to a cytotoxin.

The present invention is illustrated herein by use of murine models representative of human etiology. Evidence for this lies in the fact that (1) $CD4^-CD8^-$ cell types induce, in all systems studied, abnormal autoimmune disease causing cells and (2) type $\underline{l}$ $K^+$ channels are known to exist in humans. See Shapiro et al., Biophysical Journal 53, 550a, No. W-Pos203 (1988). By virtue of the present invention using such murine models, there has been found an established link to autoimmune diseases of elevated type $\underline{l}$ $K^+$ channels in abnormal T cells; therefore, the present invention is translatable to immune systems having CD4 and CD8 T cells bearing type $\underline{l}$ $K^+$ channels, for example, as found in humans.

Detailed Description of the Invention

The present invention is illustrated using characteristic murine models that establish, with proper correlation and translation into the human system, that type $\underline{l}$ $K^+$ channels are elevated in abnormal $CD4^-CD8^-$ T cells on onset of a given autoimmune disease. Illustrative autoimmune diseases tested were SLE, type-1 diabetes, and EAE, all with characteristic murine models. Because the present research is so properly based, given the instant disclosure of how to assay for and diagnose and treat an autoimmune disease, one skilled in the art will well enough know how to carry out such endeavors using the human system, when it is possible to proceed in this manner unhampered by regulatory impediments.

1. Brief Description of the Drawings

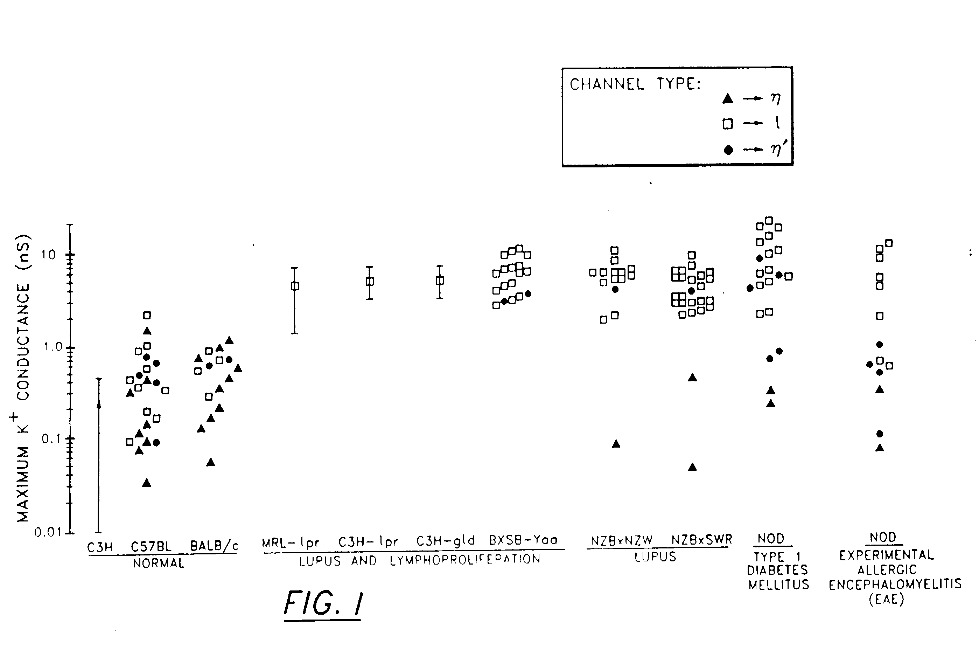

Figure 1: $K^+$ channel expression in splenic and lymph node derived $CD4^-CD8^-$Thy-$1.2^+$ T cells in normal mice and mice with SLE, type 1 diabetes mellitus, or experimental allergic encephalomyelitis (EAE; a model for multiple sclerosis).

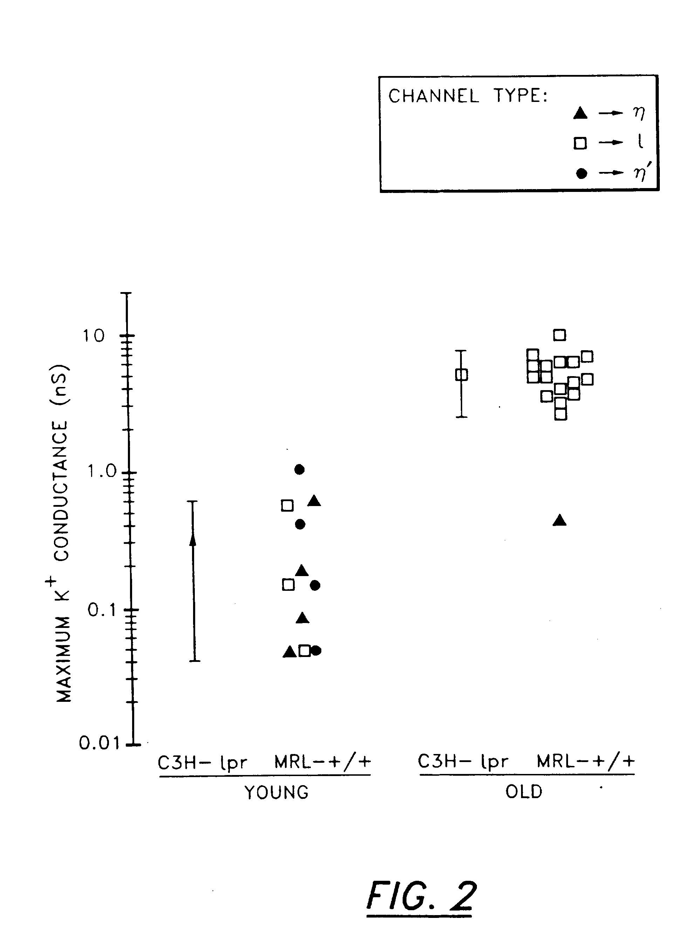

Figure 2: $K^+$ channel expression in splenic and lymph node $CD4^-CD8^-$Thy-$1.2^+$ T cells in young and old SLE mice.

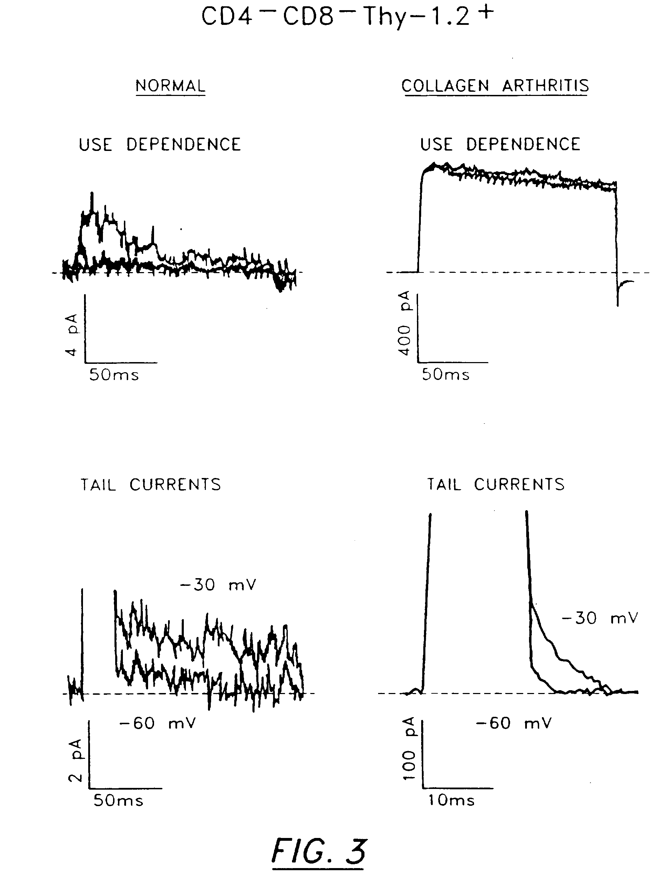

Figure 3: Whole cell $K^+$ currents in $CD4^-CD8^-$Thy-$1.2^+$ T cells in normal mice (left) and mice with collagen arthritis (right). Top, $K^+$ currents were elicited with 200 ms depolarizing pulses to 40 mV from a holding potential of -80 mV. Pulse interval was 1 s to assay cumulative (use-dependent) inactivation. Note the different current scale. Bottom, tail currents were elicited by voltage steps to -60 and -30 mV after a 15 ms prepulse to 40 mV. Note the different current and time scale.

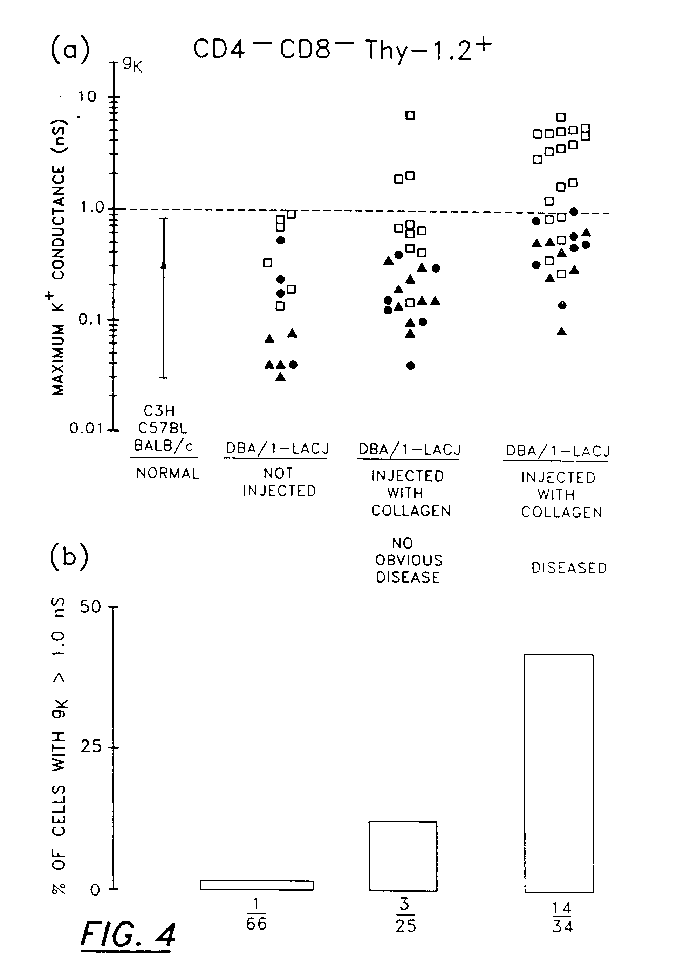

Figure 4: Maximal $K^+$ conductance ($g_K$) of CD4⁻CD8⁻Thy-1.2⁺ T cells: $\underline{a}$, , , represent $\underline{n}$, $\underline{l}$, $\underline{n'}$ K⁺ channel types. These data are shown as mean ± SD. $\underline{b}$, Percentage of cells with $g_K$ > 1000 pS of type $\underline{l}$ K⁺ conductance. Data for normal mice are pooled together from C3H, C57BL, BALB/c, and DBA/1-LACJ (uninjected).

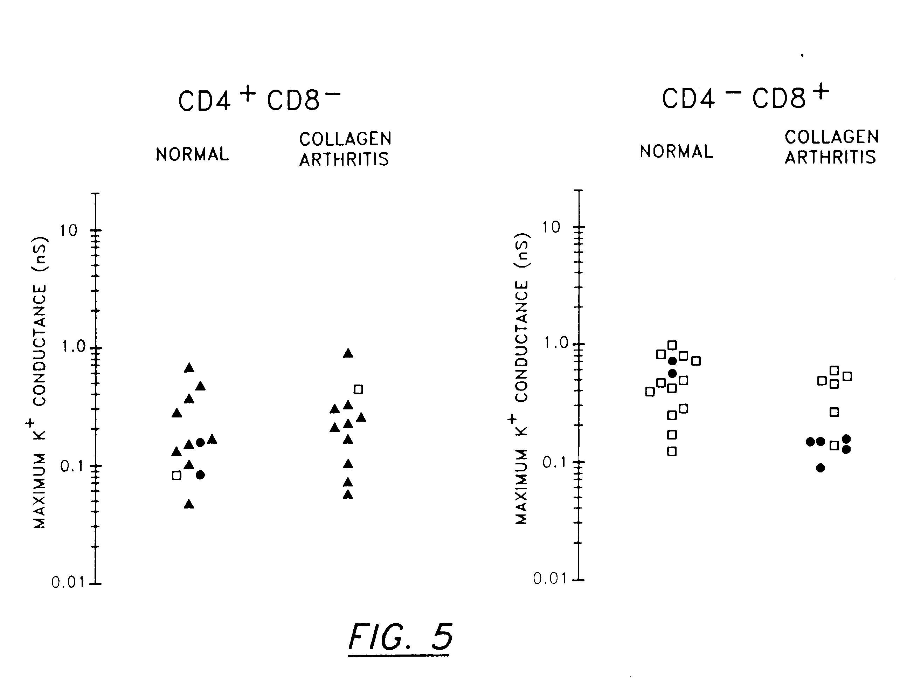

Figure 5: Maximal K⁺ conductance ($g_K$) of CD4⁺CD8⁻ and CD4⁻CD8⁺ T cell subsets in normal mice and mice with collagen arthritis. Δ, ■, • represent $\underline{n}$, $\underline{l}$, $\underline{n'}$ K⁺ channel types.

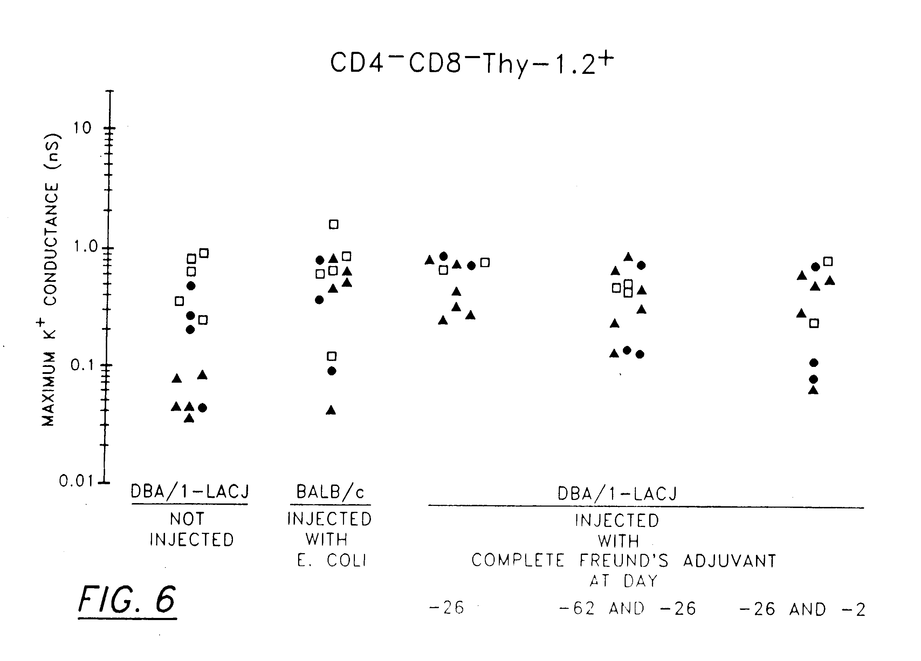

Figure 6: Maximal K⁺ conductance ($g_K$) of CD4⁻CD8⁻Thy-1.2⁺ T cells in uninjected DBA/I-LACJ mice, BALC/c mice injected with heat-killed *E. coli,* and DBA/I-LACJ mice injected with CFA at day -26, -62 and -26, -26 and -2. Day 0 refers to the day the electrophysiological experiments were performed. Δ, ■, • represent $\underline{n}$, $\underline{l}$, $\underline{n'}$ K⁺ channel types.

## 2. Definitions

By the term "extrinsic material" herein is meant any entity that is not ordinarily present or functional with respect to type $\underline{l}$ K⁺ channels and/or abnormal T cells and that affects same. Thus, the term has a functional definition and includes such identified entities as hormones; toxins; growth factors/cytokines; secondary messenger modulating substances; organic compounds and inorganic compounds; agents that indirectly impact on the $\underline{l}$ channel through effects on intracellular second messengers, or other channels, or membrane potential or receptors; cell types targeted to type $\underline{l}$ K⁺ channels or abnormal CD4⁻CD8⁻ T cells; genetic manipulation products impacting on $\underline{l}$ channels or on CD4⁻CD8⁻ T cells; or bacterial or viral materials that impact on CD4⁻CD8⁻ T cells having many l channels.

By the term "modulating effect" or "properties", or grammatical equivalents, herein is meant both active and passive impact on type $\underline{l}$ K⁺ channels and/or abnormal T cells. These include, but shall not be construed as limited to, blocking the channel or the function of the channel protein(s), reducing the number of ion channels per cell and use of secondary cell(s) or channel(s) to impact on a primary abnormal cell.

By the term "monitoring" in respect of effect of test materials on type $\underline{l}$ K⁺ channels and/or abnormal T cells herein is meant any method known or devised for measuring the impact of a test material on said channels/cells. These include, but shall not be construed as limited to, measuring current, measuring membrane potential, measuring K⁺ flux, such as with radioactive tracers, measuring K⁺ concentration and measurements of indirect consequences to other receptors, second messengers and/or channels.

The term "autoimmune disease" herein has meaning beyond the classical definition, and therefore, shall include any disease state where CD4⁻CD8⁻ T cells act as accessory cells, for example, to induce the immune system to result in destruction of organism cells.

## 3. Examples

The following examples detail materials and methods employed in the experimental procedures that follow:

Figure 1 shows the maximum K⁺ conductance, $g_K$, of CD4⁻ CD8⁻Thy-1.2⁺ T cells from normal mice and from mice with genetically determined SLE or type 1 diabetes mellitus.

A gigohm seal patch-clamp technique was employed [Hamill *et al.*, *Pflugers Arch.* 391, 85 (1981)]. Cells were separated and stained as described infra. Maximum K⁺ conductances ($g_K$) of CD4⁻CD8⁻Thy-1.2⁺ T cells were determined from the maximum peak K⁺ current elicited at +40 mV (holding potential -80 mV), assuming a reversal potential for K⁺ of -80 mV. All experiments were done at room temperature (22° to 26°C). The cells under investigation were bathed in Ringer solution (in mM): 160 NaCl, 4.5 KCl, 2 $MgCl_2$, 1 $CaCl_2$ , 2 $MgCl_2$, and 10 K-HEPES (pH 7.4). The patch pipette contained 134 KF, 11 $K_2$-EGTA, 1.1 $CaCl_2$, 2 $MgCl_2$, and 10 K-HEPES (pH 7.2). Each point represents $g_K$ in one single cell except the ones with the bars that reflect the mean±SD of previously reported data. The data from C3H (n=12), C3H-lpr (n=22), C3H-gld (n=26), and MRL-lpr (n=31) are taken from Grissmer *et al.*, *J. Immunol.* 141, 1137 (1988) and Chandy *et al.*, *Science* 233, 1197 (1986)2, respectively, and are included in the graph for comparison. Three types of voltage-gated K⁺ channels ($\underline{n}$,$\underline{l}$,$\underline{n'}$) are expressed by murine T cells, which can be distinguished by electrophysiological and pharmacological criteria. DeCoursey *et al.*, *J. Gen. Physiology* 89, 379 (1987).

$g_K$ of CD4⁻CD8⁻ T cells from three young MRL-+/+ mice, before the onset of clinically symptomatic disease and two old MRL-+/+ mice with lupus nephritis (proteinuria: >2000 mg protein/dl) was determined as described above in connection with Fig. 1. Each point represents $g_K$ in one single cell except the ones with the bars that reflect the mean±SD of previously reported data (Grissmer *et al.*, *J. Immunol.* 141, 1137 (1988) and Chandy *et al.*, *Science* 233, 1197 (1986).) See Figure 2.

Splenic CD4⁻CD8⁻Thy-1.2⁺ T cells from mice with clinically evident SLE (MRL-+/+, NZBxSWR, NZBxNAW, BXSB-males) display numerous type $\underline{l}$ K⁺ channels/cell (Figure 1) with an average $g_K$ of 4550±254 pS (mean±SEM, n=76). Dividing the $g_K$ by the single channel conductances [Lewis *et al.*, *Science* 239, 771 (1988)] gives an estimate of 100-200 type l K⁺ channels. Previous data on K⁺ channel expression in CD4⁻CD8⁻Thy-1.2⁺ T cells from C3H-lpr ($g_K$: 5223±565 pS; mean±SEM, n=22), C3H-gld ($g_K$: 5092 ±503 pS; mean±SEM, n=26), MRL-lpr ($g_K$: 4600±600pS; mean±SEM, n=31) mice [Grissmer *et al.*, *J. Immunol.* 141, 1137 (1988) and Chandy *et al.*, *Science* 233, 1197 (1986)], is presented for comparison. Occasionally, cells with small numbers of type $\underline{n}$ K⁺ channels were observed; these may represent normal cells. In contrast, CD4⁻CD8⁻Thy-1.2⁺ T cells from the spleen and lymph nodes of normal mice (C57BL, BALB/c), varying in age from 1-19 months, express types $\underline{n}$, I or $\underline{n}'$ K⁺ channels, with a low average $g_K$ of 458±63 pS (mean±SEM, n=39) representing about 10-20 K⁺ channels/cell. Thus, CD4⁻CD8⁻Thy-1.2⁺ T cells from every murine model for SLE, display an abnormally elevated number of type $\underline{l}$ K⁺ channels, demonstrating from the (as labeled) Lupus models that the alteration is linked to the disease.

Besides the murine SLE models, the non-obese diabetes (NOD) mouse strain is another genetically-determined murine model for autoimmune disease [Makino et al., Exp. Anim. 29, 1 (1980) and Hattori et al., Science 231, 733 (1986)]. These mice spontaneously develop type 1 diabetes mellitus. CD4⁺CD8⁻ and CD4⁻CD8⁺ T cells have been implicated in the pathogenesis of this disease [Miller et al., Nature 307, 465 (1984), O'Neil et al., J. Immunol. 140, 52 (1988) and Koike et al., Diabetes 36, 539 (1987)]. The role of CD4⁻CD8⁻Thy-1.2⁺ T cells has not been examined. Splenic CD4⁻ CD8⁻Thy-1.2⁺ T cells from NOD mice with clinical evidence of type 1 diabetes mellitus, exhibited large numbers of type $\underline{1}$ K⁺ channels, with an average $g_K$ of 5795±1253 pS (mean±SEM, n=21), reflecting about 200 type $\underline{l}$ K⁺ channels/cell. This pattern of K⁺ channel expression is almost identical to that in CD4⁻CD8⁻Thy-1.2⁺ T cells from SLE mice, showing a common mechanism underlying type 1 diabetes mellitus and SLE. A relatively larger fraction of the splenic CD4⁻CD8⁻ T cells (421) in mice with type 1 diabetes mellitus, displayed lower $g_K$ than observed in SLE mice (4/76). This difference in K⁺ channel expression in the spleen may reflect the organ-specific nature of the autoimmune manifestations in type 1 diabetes mellitus, which are primarily confined to the pancreas, and the systemic autoimmune manifestations in SLE.

Since murine SLE and type 1 diabetes mellitus are genetically determined diseases, alteration in K⁺ channel expression may be present from birth. To address this question a comparison of K⁺ channel expression in CD4⁻CD8⁻Thy-1.2⁺ T cells from young and old MRL-+/+ mice was undertaken. CD4⁻CD8⁻Thy-1.2⁺ T cells from young MRL-+/+ mice displayed an order of magnitude fewer K⁺ channels ($g_K$: 295±91 pS; mean±SEM, n=11; ~10 channels/cell) compared to older MRL-+/+ ($g_K$: 5306±518 pS; mean±SEM, n=18; ~200 channels/cell) with clinical evidence of lupus nephritis (Figure 2). Earlier data on CD4⁻CD8⁻Thy-1.2⁺ T cells from young healthy C3H-lpr ($g_K$: 324±67 pS; mean±SEM, n=17; ~12 channels/cell) mice are presented for comparison. The pattern of K⁺ channel expression in autoimmune mice, prior to the onset of clinically symptomatic disease, resembles that in CD4⁻CD8⁻Thy-1.2⁺ T cells from normal mice (compare Figs. 1 and 2). These data demonstrate that abnormal K⁺ channel expression in CD4⁻CD8⁻ T cells parallels the development of disease and reflects the ability of these cells to induce the secretion of pathogenic anti-DNA autoantibodies.

Experimental allergic encephalomyelitis (EAE) is considered to be a model for human multiple sclerosis. Mice inoculated with myelin basic protein develop paralysis within 1-2 weeks (acute EAE). Some of these mice recover and then develop a chronic relapsing disease (chronic EAE) which closely resembles human multiple sclerosis. CD4⁻CD8⁻ Thy1.2⁺ splenic T cells were isolated from mice with chronic EAE and their K⁺ channel expression was studied. A greater frequency of cells exhibited large numbers of type $\underline{l}$ K⁺ channels compared to normal mice. This pattern of K⁺ channel expression is similar to that in CD4⁻CD8⁻Thy1.2⁺ from mice with SLE and type-I diabetes mellitus, showing a common mechanism underlying these three distinct autoimmune diseases. A larger fraction of CD4⁻CD8⁻Thy1.2⁺ cells had normal numbers of K⁺ channels/cell compared with mice with SLE. The autoimmune process in chronic EAE is primarily confined to the nervous system and changes in the spleen would not be as evident as in SLE which is primarily a systemic autoimmune disease with a major autoimmune process taking place in the spleen.

Other subsets of T lymphocytes (CD4⁺CD8⁻ or CD4⁻CD8⁺) from diseased BXSB-male mice and from C3H-lpr, C3H-gld mice exhibited small numbers of types $\underline{l}$, $\underline{n}$, or $\underline{n}'$ K⁺ channels like phenotypically identical cells from normal mice. Normal mitogen activated T cells express ~200 type $\underline{n}$ K⁺ channels/cell [DeCoursey et al., J. Gen. Physiol. 89, 405 (1987)], in contrast to the numerous type $\underline{l}$ K⁺ channels displayed by CD4⁻CD8⁻Thy-1.2⁺ T cells in mice with SLE and type 1 diabetes mellitus. These data demonstrate that abundant type $\underline{l}$ K⁺ channel expression is an unique feature of diseased CD4⁻CD8⁻ T cells, and is different from the pattern of K⁺ channel expression in normal T cells induced by mitogens.

K⁺ channels were identified on the basis of their inactivation properties, channel closing kinetics, and sensitivity to block by tetraethylammonium (TEA⁺). Typically type $\underline{n}$ K⁺ channels are use-dependent, i.e., show cumulative inactivation by repetitive depolarizing pulses. Use dependence was determined by measuring the decline in the size of the peak K⁺ current elicited with one per second repetitive depolarizing pulses of 200 ms duration. Type $\underline{n}$ K⁺ channels also close slowly upon repolarization with a time constant of about 30 ms at -60 mV and are blocked by TEA⁺ ($K_D$ = 8 mM). Type $\underline{l}$ K⁺ channels are not use dependent, close more rapidly on repolarization with a time constant of 2 ms at -60 mV, and are much more sensitive to block by TEA⁺ ($K_D$ = 0.1 mM). Type $\underline{n}'$ K⁺ channels are not use dependent, close slowly like type $\underline{n}$ K⁺ channels upon repolarization, but are less sensitive to lock by TEA⁺ ($K_D$ ~100 mM).

For the above experiments, mice were housed in the Vivarium at U.C. Irvine, and care was taken in accordance with institutional guidelines. The mice were lightly anesthetized with metofane, and sacrificed by cervical dislocation. The spleen was removed and single cell suspensions prepared. Cells were washed three times with RPMI-1640 (Irvine Scientific, Santa Ana, CA) supplemented with 10% fetal calf serum (Hyclone, Logan, Utah). Cells were resuspended at 10 x 10$^6$ cells/ml and T cells separated from non-T cells by a nylon wool column. The cells were washed three times in medium, and resuspended at 1-2 x 10$^6$ cells/ml. The cells were stained as follows: Cells were first incubated on ice for 30 minutes with rat-anti-mouse-CD4 and rat-anti-mouse-CD8 antibodies, washed three times in medium, incubated for 30 minutes on ice with phycoerythrin-conjugated goat-anti-rat-IgG (affinity purified and absorbed for mouse Ig), washed three times in medium, incubated on ice for 30 minutes with fluoresceinated rat-anti-mouse-Thy-1.2, washed three times in medium, and then resuspended at 2 x 10$^6$ cells/ml in medium. The cells were then patch-clamped as described in the Fig. 1 legend. CD4$^-$CD8$^-$Thy-1.2$^+$ T cells were identified as green cells. Other T cells (CD4$^+$CD8$^-$Thy-1.2$^+$, CD4$^-$CD8$^+$Thy$^-$1.2$^+$,) were yellow, and B cells and monocytes (CD4$^-$CD8$^-$Thy-1.2$^-$) were unstained.

The mice used were: C57BL: 6 and 19 months; BALB/c: 1 and 7 months; BXSB-males: 2 mice aged 4 months; NZBxNZW: 2 mice aged 6 months, one aged 9 months; NZBxSWR: 3 mice aged 7-8 months; old MRL-+/+: 2 mice older than 20 months; NOD mice: 6 and 9 months; young MRL-+/+: 6, 10, and 12 months.

A. Assay for screening drugs

1) Studies on mouse lymphocytes

a) Sacrifice of mice: Mice with autoimmune diseases (for example, systemic lupus erythematosus or type-1 diabetes mellitus) are sacrificed humanely by cervical dislocation after light anesthesia with metofane.
b) Isolating T cells from spleen, thymus or lymph nodes: The spleen, thymus, and lymph nodes are removed from the animal and single cell suspensions prepared by crushing the organ with the barrel of a syringe through a wire mesh. The cells are then washed three times with a cell sustaining medium, for example RPMI-1640 medium supplemented with heat inactivated fetal calf serum (10%), 1-glutamine (1 mM), and appropriate antibiotics (for example penicillin/streptomycin). The cells are then counted and resuspended at a concentration of 10 x 10$^6$ cells/ml. 50 x 10$^6$ cells are then placed on a nylon wool column to separate T cells from other blood cells as described previously in Grissmer et al. J. Immunol. 41, 1137 (1988).
e) Staining: The T cells are incubated with anti-CD4 and anti-CD8 antibodies in the cold for an appropriate period (for example, 20-30 minutes), washed in the RPMI-1640 medium described Supra, incubated in the cold with an appropriate dye-conjugated second antibody (for example, phycoerythrin-conjugated goat-anti-rat IgG antibody), washed in the medium described Supra, and incubated in the cold with an appropriate dye-conjugated T cell specific antibody (for example fluorescein-conjugated anti-Thy-1.2, or anti-CD3, or anti-T cell receptor), washed with medium described Supra, and then resuspended at 1-5 x 10$^6$ cells/ml in medium, and used for the experiments described below. The CD4$^-$CD8$^-$ T cells are then identified under the fluorescence microscope as cells expressing T cell-specific molecules like Thy-1.2, CD3 or T cell receptors, and not expressing the CD4 or CD8 molecules.

2) Studies on other animal systems
Similar approaches are used to isolate, identify and evaluate for exploitation according to this invention CD4$^-$ CD8$^-$ T cells in other animal models, including humans, that express the CD4 and CD8 molecules in the immune system.
3) Studies on human cells

a) Separation of cells from blood: Blood from patients with autoimmune diseases, for example, systemic lupus erythematosus are collected. Mononuclear cells are separated from the blood on a density gradient, for example, Ficoll®-Hypague. The mononuclear cells are washed with the medium described Supra, and T cells isolated either by the nylon wool column method described Supra, or by rosetting with sheep red blood cells as described in Chandy et al. J. Exp. Med. 160, 369 (1984) or by any other standard method available. T cells are similarly isolated from available lymphoid tissues.
b) Staining: The cells are stained as described Supra, first with anti-CD4 and anti-CD8, followed by an appropriate dye-conjugated second antibody, for example, phycoerythrin-conjugated goat-anti-mouse IgG antibody, followed by an appropriate dye-conjugated T-cell-specific antibody, for example, fluorescein-conjugated anti-CD3, or anti-CD2, or anti-T cell receptor, or anti-Leu-2. The CD4$^-$CD8$^-$ T cells are then identified under the fluorescence microscope as cells expressing T-cell-specific molecules like CD3 or CD2 or Leu-1 or T cell receptors, and not expressing the CD4 or CD8 molecules.

4) Electrophysiological recordings:
The cells are placed into a recording chamber and CD4$^-$CD8$^-$ T cells identified appropriately with an inverted microscope under fluorescent light, as described Supra. In cases where the predominant population of T cells is CD4⁻CD8⁻, for example, in lpr and gld mice or CD4⁻CD8⁻ cell lines, staining may not be required to identify the cells. These cells are studied electrophysiologically using the patch-clamp technique (Hamill et al. Pflugers Arch. 391, 85 (1981).

a) Solutions: The cells under investigation are bathed in normal mammalian Ringer solution (160 mM NaCl, 4.5 mM KCl, 2.0 mM CaCl$_2$, 1.0 mM MgCl$_2$, 5 mM HEPES, adjusted to pH 7.4 with NaOH; 290 to 320 mosm). Other cell-sustaining solutions can also be used. The patch pipette contains an appropriate solution, for example: 134 mM KF, 11 mM EGTA, 1 mM CaCl$_2$, 2 mM MgCl$_2$, 5 mM HEPES, adjusted to pH 7.2 with KOH; 285 to 310 mosm.

b) Identification of type l K⁺ channels: By varying the voltage across the membrane, voltage-gated ion channels are opened and ion movement through these channels is measured as ionic current. Several distinct channel types have been characterized using a variety of electrophysiological techniques (Hille B: Ionic channels of excitable membranes, Sinauer Associates, Sunderland, MA (1984). These channels are distinguished on the basis of electrophysiological and pharmacological criteria. Type l K⁺ channels are identified in the patch-clamped cells on the basis of their inactivation properties, channel-closing kinetics, sensitivity to block by TEA⁺, single channel amplitude, and voltage dependence of activation as described in detail by DeCoursey et al. J. Gen. Physiol 89, 379 (1987).

c) Drug testing: After having identified the l type K⁺ channels, the bathing solution is changed and replaced by a solution containing the drug of interest. The effect of the drug on the type l K⁺ channels is monitored electrophysiologically, for example, changes of inactivation properties, or channel closing kinetics, or block, or shift in voltage dependence or activation, or alteration of single channel amplitude, etc. The same procedures are used to screen a battery of test materials on the same cell or on other cells containing type l K⁺ channels. The selectivity of this drug to type l K⁺ channels is determined by testing the effect of these test materials on other ion channel types, for example, type n K⁺ channels in T cells. Drugs that modulate type l K⁺ channels selectively and with great potency are identified.

d) Preparation of antibodies against type l K⁺ channels: The genes encoding type l K⁺ channels are isolated by standard recombinant DNA techniques such as described in Weir et al., Handbook of Experimental Immunology, Vol. 3 (1986) and other available documents. These genes are used as templates to prepare type l K⁺ channel proteins or peptides, which are used as antigens to prepare antibodies against type l K⁺ channels. A second method for preparing antibodies against type l K⁺ channel is used with cells expressing large numbers of type l K⁺ channels, isolating the cell surface proteins of these cells and using these proteins as antigens for the preparation of antibodies. The antibodies are screened for their ability to (a) effect type l K⁺ channels electrophysiologically, as described Supra, or (b) for their ability to destroy cells expressing type l K⁺ channels, when the antibodies are conjugated to a cell toxin, or when the antibodies bind to the cell in the presence of complement, or (c) for the ability of radioisotope-, or dye-, or enzyme-linked antibodies to attach to the cells, attachment being monitored by fluorescence microscopy, by fluorescence cell sorting, by radioactive counting, or by enzyme-linked immunosorbent assays, or other appropriate techniques.

e) Drug and/or antibody testing in autoimmune disease: Drugs or antibodies identified by the assays described Supra as being selective for type l K⁺ channels are testing in vivo for efficacy in appropriate animal models, for example, for their ability to retard the onset and development of autoimmune diseases, or reverse autoimmune diseases. The route of administration of the drugs/antibodies can be oral, parenteral, or via the rectum, and the drug could be administered alone as principals, or in combination with other drugs or antibodies, and at regular intervals or as a single bolus, or as a continuous infusion in standard formations. Drugs or antibodies described supra are also tested in in vitro assays, for example, for their ability to stimulate B cells from autoimmune patients or animal models to secrete autoantibodies.

f) A treatment protocol: Drugs or antibodies identified by the assays described above are tested for safety in humans as per Federal guidelines. These drugs or antibodies described supra are administered via standard formulations to patients with autoimmune diseases, again either orally, parenterally, rectally, alone or in combination, at regular intervals or as a single bolus, or as a continuous infusion, for modulating type l K⁺ channels in the CD4⁻CD8⁻ T cells thereby abrogating their abnormal accessory helper function and impacting on the course of the autoimmune disease.

B. Other Murine Autoimmune Disorders:

Mice

DBA-J/Lac-J male mice were purchased from the Jackson Laboratory (Bar Harbor, ME). They were injected intradermally near the base of their tails with 100ug of highly purified chick type II collagen emulsified in complete Freunds Adjuvant (CFA) that contained additional Mycobactrium butyricum (Difco). The collagen/CFA emulsion was prepared by adding 1 volume of modified CFA (30 ug of M. butyricum per 50 ul of 0.01 N acetic acid). Animals were immunized with 100 ul of this mixture using a 26 gauge needle. One group of control animals was not injected and another group was injected with modified CFA without collagen. Paw thickness was measured with a Schnelltaster constant-tension caliper. The type II collagen was prepared from chick sternal cartilage by established methods Miller, Biochemistry 10, 1652 (1971).

E. coli (ATCC 8739, wild type) was homogenized in a Manton-Gaulin homogenizer at $4,19.10^8$ Pa (6000 psi) for 5 min, and heatinactivated for 30 min at 60°C. The bacteria were then adjusted to a concentration of 0.5 mg/ml stock in phosphate buffered saline (PBS). BALB/c mice were then immunized intraperitoneally with 1 ml of solution containing 0.5 ml antigen (15 ug. E. Coli) in PBS and 0.5 ml CFA. Mice received booster injections of antigen (15 ug E.Coli) and incomplete Freund's adjuvant 4 weeks and 6 weeks later. Mice were sacrified 1 week after the second booster immunization.

(2) Antibodies: PE-conjugated-anti-CD4 (L3T4), FITC-conjugated-anti-CD8, FITC-conjugated-anti-Thy-1,2, anti-CD4 and anti-CD8 were purchased from Becton Dickinson (Mountain View, CA). PE-conjugated-goat-anti-rat IgG (affinity purified and absorbed against mouse IgG) were purchased from Caltag (Rupp and Bowman, Tustin, CA).

(3) Separation of T Cells: Mice were killed, and single-cell suspensions were prepared from the spleen. T cells were enriched by passage through a nylon wool column. Cells were then suspended in RPMI-1640 medium supplemented with 10% heat-inactivated FCS (Hyclone, Logan, UT) and 2 mM l-glutamine (medium).

(4) Staining: Cells ($2\times10^6$) were incubated with an appropriate dilution of anti-CD4-PE and anti-CD8-FITC for 30 min on ice, washed three times with medium, and resuspended in 1 ml of medium. The stained cells were plated into glass chambers, and the $CD4^+CD8^-$ (appears orange) and $CD4CD8^+$ (green) were identified by epifluorescence microscopy. To identify DN T cells, cells were incubated with anti-CD4 and anti-CD8 for 30 min on ice, washed three times with medium, incubated with PE-labeled-goat-anti-rat IgG for 30 min on ice, washed five times with medium incubated with FITC-labeled-anti-Thy-1.2 for 30 min on ice, washed three times with medium, and then resuspended in 1 ml of medium. The cells were visualized under the microscope and three populations were evident: $CD4^-CD8^-Thy-1.2^+$ cells appeared green, $CD4^+CD8^-Thy-1.2^+$ and $CD4^-CD8^+Thy-1.2^+$ cells appeared yellow, and B cells and macrophages were unstained. These staining protocols do not affect channel expression (Chandy, et al. Eur. J. Immunol. (in press); Lewis and Cahalan (1988), Science 239, 771); Grissmer, et al. (1988), J. Immunol. 141, 1137). In most experiments, chambers were coated with polylysine (0.25 mg/ml) to improve cell adherence to the dish. This procedure did not alter channel expression when compared with cells plated into uncoated chambers.

(5) Electrophysiology: After phenotypic identification by epifluorescence -microscopy, single T cells were patch-clamped at room temperature (22° to 26° C). Details of the giga-ohm voltage-clamp technique used here are described elsewhere (Chandy, et al. Eur. J. Immunol. (in press); Lewis and Cahalan (1988), Science 239, 771); Grissmer, et al. (1988) J. Immunol. 141, 1137); Chandy, et al. (1986) Science 233, 1197; Cahalan, et al. (1985) J. Physiol. 358, 197.)

Solutions: The cells under investigation were bathed in normal mammalian Ringer solution containing (in mM): 160 NaCl, 4.5 KCl, 2 $MgCl_2$, $^1CaCl_2$, and 5 Na-HEPES (pH 7.4). The patch pipette contained 134 KF, 11 $K_2$-EGTA, 1.1 $CaCl_2$, 2 $MgCl_2$ and 10 K-HEPES (pH 7.2). In Ringer solutions containing tetraethylammonium chloride ($TEA^+$), NaCl was replaced by the appropriate $TEA^+$ concentrations keeping the osmolarity constant. The bath solution could be changed during recordings by bath perfusion.

Identification of $K^+$ channel type: $K^+$ channels were identified on the basis of their inactivation properties, channel closing kinetics, and sensitivity to block by $TEA^+$ (Chandy, et al. Eur. J. Immunol. (in press) ; Lewis and Cahalan (1988), Science 239, 771; Grissmer, et al. (1988), J. Immunol. 141, 1137; Chandy, et al. (1986), Science 233, 1197; DeCoursey, et al. (1987), J. Gen. Physiol. 89, 379; DeCoursey, et al. (1987), J. Gen. Physiol. 89, 405). Type n $K^+$ channels are use-dependent, close slowly upon repolarization with a time constant of about 30 ms at -60 mV and are blocked by $TEA^+$ ($K_D$ = 8mM). Type l $K^+$ channels are not use-dependent, close more rapidly on repolarization with a time constant of 2 ms at -60mV, and are much more sensitive to block by $TEA^+$ ($K_D$ = 0.1 mM). Type n' $K^+$ channels are not use-dependent, close slowly as do type n $K^+$ channels upon repolarization, but are less sensitive to block by $TEA^+$ ($K_D$ - 100 mM).

Determination of maximal $K^+$ conductance ($g_K$) and number of $K^+$ channels per cell. $g_K$ was calculated from the largest $K^+$ current recorded in each cell. A reversal potential of -80 mV was used to calculate $g_K$ (Cahalan, et al., J. Physiol. 358, 197 (1985). The number of $K^+$ channels per cell was calculated by dividing $g_K$ by the single-channel conductances of the appropriate channel type; the single-channel conductances are 18, 27, and 17 pS for n, l and n', respectively (Chandy, et al. Eur. J. Immunol. (in press); Lewis and Cahalan (1988), Science 239, 771); Grissmer, et al. (1988) J. Immunol. 141, 1137).

RESULTS

<u>DN T cells from mice with collagen arthritis possess abnormally large numbers of type 1 K$^+$ channels.</u> Fig. 3 shows K$^+$ outward currents in DN T cells from normal DBA/1-LacJ mice and mice with collagen arthritis. In the experiments shown in the upper panel, the decline in the size of the K$^+$ current elicited by a repetitive (1/s) depolarizing voltage-step from -80 mV to +40 mV for a duration of 200 ms was measured. This property of K$^+$ channels which is termed use dependence is characteristic of type <u>n</u> K$^+$ channels, but not of type l or <u>n'</u>. Another feature of type <u>n</u> K$^+$ channels is their closing kinetics during membrane hyperpolarization, after an initial period of depolarization. This property called deactivation or tail currents is shown in the bottom panel of Fig. 3. In these studies, the membrane potential was held at -80 mV, then stepped to +40 mV for a 15 ms duration to open all the channels, and then stepped back to either -30 or -60 mV. Type <u>n</u> and <u>n'</u> channels close slowly (time constants for closure are 75 ms and 40 ms at -30 mV and -60 mV, respectively), whereas the l K$^+$ channels close rapidly (time constants for closure are 6 ms and 2 ms at -30 mV and -60 mV, respectively.

Normal DN T cells (left panel of Fig. 3) have small K$^+$ currents that are use-dependent (top), display slow deactivation kinetics (bottom), are half-blocked by 10 mM TEA$^+$ (not shown), indicating that they express small numbers of type <u>n</u> K$^+$ channels. DN T cells from diseased mice have large K$^+$ currents (right panel of Fig. 3) that are not use dependent (top), exhibit rapid deactivation kinetics (bottom), are half-blocked by 0.1 mM TEA$^+$ (not shown), indicating that these cells express an abundance of type l K$^+$ channels.

Fig. 4a shows the maximum K$^+$ conductance or $g_{Kmax}$, of splenic DN T cells from normal and diseased mice, and Fig. 4b represents the fraction of cells with large numbers of type l K$^+$ channels. The upper limit for the $g_K$ of DN T cells from normal DBA/l-LacJ mice, is 1000 picosiemen (pS), with an average of $299 \pm 78$ pS. The $g_K$ of DN T cells from three other normal strains of mice (C3H-HeJ, BALB/c and C57BL) are shown for comparison ($g_K = 405 \pm 53$ pS; mean $\pm 53$ pS; mean $\pm$ SEM; n = 51). These data indicate that normal DN T cells express roughly 10-20 K$^+$ channels/cell of types <u>n</u>, l or <u>n'</u>. In marked contrast, 14 out of 34 DN T cells from diseased DBA/1-LacJ mice immunized with type II collagen, exhibited a $g_K$ greater than 1000 pS (Fig. 4a and 4b), and the channels in these cells were exclusively type l, averaging 180 channels/cell. Interestingly, DN T cells exhibiting large numbers of type l K$^+$ channels were substantially fewer (3/25) in DBA/l-LacJ mice immunized with type II collagen with no obvious clinical signs of arthritis (Fig. 4a and 4b) compared with mice with obvious disease. These data show that elevation of type l K$^+$ channel expression in DN T cells parallels the development of collagen arthritis.

<u>Other T-cell subsets from diseased mice retain their normal pattern of expression.</u> Fig. 5 shows the $g_K$ of helper (CD4$^+$CD8$^-$) and cytotoxic (CD4$^-$CD8$^+$) T cells from normal and diseased mice. CD4$^+$CD8$^-$ T cells from both normal and diseased mice exhibited small number of K$^+$ channels, (averaging 10-20 channels per cell), which were predominantly type <u>n</u>. CD4$^-$CD8$^+$ T cells from mice with collagen arthritis displayed small numbers of types <u>n'</u> or l K$^+$ channels like their phenotypic counterparts from normal mice. Thus, the augmentation of type l K$^+$ channel numbers associated with disease, appears to be a feature confined to DN T cells.

<u>Abundant type l K$^+$ channel expression in DN T cells is not a feature of a generalized immune response in vivo.</u> To discern whether the enhanced numbers of type l K$^+$ channels in DN T cells were unique to autoimmune disorders, or whether it reflected a generalized immune response <u>in vivo,</u> DN T cells were examined from mice immunized with either heat-killed <u>E. Coli</u> or CFA (Fig. 6). Mice received either one CFA immunization (26 days before patch=-clamp experiments) or booster doses of CFA either 62 or 26 days before or 26 and 2 days before patch-lamp experiments. ND T cells from these mice possessed small numbers of types n, l or <u>n'</u> channels, regardless of whether they received one immunization or booster doses. Since T cells activated by mitogens <u>in vitro</u>, enlarge (have an average membrane capacitance > 2 pF corresponding to a cell diameter > 8 um), we compared $g_K$ of large (> 2 pF) and small (< 2 pF) DN T cells in CFA immunized mice. $g_K$ of large cells ($580 \pm 170$ pS; mean $\pm$ SD; n = 5) was not significantly different from that of small cells ($500 \pm 275$ pS; mean $\pm$ SD; n = 27). Similarly, DN T cells from mice immunized with heat-killed <u>E. Coli</u> also display small numbers of K$^+$ channels of one of the three types. Taken together these data show that expression of a high number of type l K$^+$ channels by DN T cells is associated with symptoms of autoimmune diseases.

Studies with the patch clamp recording technique have revealed the presence of three distinct types of voltage-gated K$^+$ channels in murine T cells (Chandy, et al. Eur. J. Immunol. (in press); Lewis and Cahalan (1988), Science 239, 771); Grissmer, et al. (1988) J. Immunol. 141, 1137); Chandy, et al. (1986) Science 233, 1197). These channels termed <u>n</u>, <u>n'</u> and l are distinguishable on the basis of biophysical and pharmacological criteria. Helper and cytotoxic T cells can be differentiated on the basis of their pattern of K$^+$ channel expression. CD4$^+$CD8$^-$ (helper) T cells display about 20-100 type <u>n</u> K$^+$ channels whereas CD4$^-$CD8$^+$ (cytotoxic) T cells exhibit 20-200 type l K$^+$ channels (Chandy, et al. Eur. J. Immunol. (in press); Lewis and Cahalan (1988), Science 239, 771; Grissmer, et al. (1988) J. Immunol. 141, 1137). Mitogen-activated T cells express about 20-times more K$^+$ channels than unstimulated cells, which are exclusively type <u>n</u> (22). Quiescent DN T cells possess small numbers of one of these types of channels (Chandy, et al. Eur. J. Immunol. (in press), Grissmer, et al. (1988) J. Immunol. 141, 1137). Abundant type $I$ K$^+$ channel expression is a marker for DN T cells associated with murine SLE, type-1 diabetes mellitus or chronic EAE; other T-cell subsets from mice with these autoimmune diseases retain their normal pattern of K$^+$ channel expression. Here, we have extended our observations to mice with type II collagen arthritis, an autoimmune disorder that shares many immunological features with SLE, type 1 - diabetes and chronic EAE.

A substantially large fraction of DN T cells, from mice and active type II collagen arthritis, display elevated numbers of type $I$ K$^+$ channels. In contrast, phenotypically similar cells from normal mice (Fig. 3 and 4), or from mice immunized with CFA or heat-killed E. Coli (Fig. 6), or mice inoculated with type II collagen without evidence of active disease, exhibit small numbers of K$^+$ channels which may be types $n$, $n'$ or $I$. The pattern of K$^+$ channel expression in helper (CD4$^+$CD8$^-$) and cytotoxic (CD4$^-$CD8$^+$) T cells from arthritic mice closely resemble that of their phenotypic equivalents in normal strains of mice. Thus, altered K$^+$ expression appears to be a marker for DN T cells associated with four disparate autoimmune disorders.

Recent reports show that gamma/delta DN T cells respond to mycobacterial antigens and accumulate in leprosy skin lesions, cutaneous leishmaniasis, and rheumatoid arthritic joints (Modlin, et al. (1989), Nature 339, 544); Holishitz, et al. (1989), Nature 339, 226). By inducing the aggregation of monocytes, these DN T cells may contribute to inflammatory processes (Modlin, et al. (1989), Nature 339, 544). Alpha/beta TCR$^+$ DN T cells have been reported to act as helper cells, inducing autoreactive B cells to secrete pathogenic Anti-DNA antibodies (Datta, et al. (1987), J. Exp. Med. 165, 1252); Sainis and Datta (1988), J. Immun. 140, 2215). DN T cells have also been reported to abrogate oral tolerance (Kitamura, et al. (1987), J. Immunol. 139, 3251). Collectively, these observations show that DN T cells apparently have a significant role in biologically relevant immune responses and apparently are involved in the mechanisms that result in tissue damage found in autoimmune diseases. Activation via a pathway distinct from that triggered by mitogens or antigens (e.g., E. coli or CFA), may induce abundant expression of type $I$ K$^+$ channels on DN T cells, regardless of the type of TCR they display on their cell surface.

## Claims

1. An assay for screening and identifying extrinsic materials having a modulating effect on type $I$ K$^+$ channels of abnormal double-negative (CD$_4^-$CD$_8^-$). T cells exhibiting excess $I$ K+ channels linked to an auto immune disease comprising the steps comprising:

   a) providing a culture of abnormal double-negative T cells, abnormal by their exhibiting unique K$^+$ channel alteration characteristic of and linked to an autoimmune disease characterized by elevated numbers of type $I$ K$^+$ channels,
   b) contacting said culture of cells with one or more of a battery of test materials that can potentially modulate the type $I$ K$^+$ channels thereof,
   c) monitoring the effect of said test materials on said type $I$ K$^+$ channels, and
   d) selecting candidates from the battery of test materials capable of modulating type $I$ K$^+$ channels.

2. An assay according to Claim 1 wherein the monitoring of step c.) is conducted by measuring the electrical current through the K$^+$ channels bathed with said test material.

3. An assay according to Claim 2 wherein the selecting of step d.) is based upon test material inducing little or less than normal electrical current through the type $I$ K$^+$ channels or upon changing the intensity of a membrane potential sensing dye or a calcium sensing dye.

4. An assay for determining presence of an auto immune disease state in an individual comprising the steps comprising :

   a) providing T cells containing K$^+$ ion channels from a test individual,
   b) identifying abnormal double-negative T cells exhibiting excess type $I$ K$^+$ channels from among the population of T cells of step a),
   c) measuring the relative numbers of type $I$ K$^+$ channels of the cells identified in step b), and
   d) determining whether said type $I$ K$^+$ channels are elevated over normal.

5. The assay according to Claim 4 wherein the measuring of step c.) is conducted by measurement of electrical current through the type $I$ K$^+$ channels.

6. The assay according to Claim 5 wherein the determining of step d) is based upon measurement of electrical current through type $I$ K$^+$ channels or upon the intensity of dye staining in a normal phenotypically similar T cell.

7. Use of an extrinsic material for the preparation of a drug useful for treating an autoimmune disease, the said extrinsic material having a modulating effect on type $I$ K$^+$ channel in abnormal double-negative T cells exhibiting excess type $I$ K$^+$ channels linked to an autoimmune disease.

**8.** Use according to Claim 7 wherein said material is conjugated with a cytotoxin and has an abnormal double-negative T cell type *I* specific antibody to locate said abnormal double-negative T cells.

**9.** Use according to Claim 7 wherein said material is tetraethyl ammonium.

**10.** Use according to Claim 7 wherein said autoimmune disease is systemic lupus erythematosus.

**11.** Use according to Claim 7 wherein said autoimmune disease is type 1 diabetes mellitus.

**12.** Use according to Claim 7 wherein said autoimmune disease is collagen rheumatoid arthritis.

**13.** Use according to Claim 7 wherein said autoimmune disease is multiple sclerosis (EAE encephalomyelitis).

**14.** Use according to Claim 7 wherein said autoimmune disease is myasthenia gravis.

**15.** Use according to Claim 7 wherein said material is a candidate identified according to the assay as defined in Claim 1.

**16.** A process which comprises compounding a candidate selected according to the assay as defined in Claim 1 in the preparation of a composition containing said candidate as an essential component, said composition being useful to impart its modulating properties when it is contacted in vivo with immune systeme abnormal double-negative T cells containing excess type *I* K$^+$ channels.

**17.** Use of a composition obtained by the process as defined in Claim 16 for the preparation of a drug for treating an autoimmune disease.

**Patentansprüche**

**1.** Assay zum Screenen und Identifizieren extrinsischer Materialien mit einer Modulationswirkung auf Typ-I-K$^+$-Kanäle von abnormalen doppelt-negativen (CD$_4^-$CD$_8^-$) T-Zellen, die einen Überschuß an I-K$^+$-Kanälen zeigen, verbunden mit einer Autoimmunkrankheit, umfassend die Stufen:

a) Schaffung einer Kultur aus abnormalen doppelt-negativen T-Zellen, abnormal weil sie einzigartige K$^+$-Kanaländerungseigenschaften zeigen von einer Autoimmunkrankheit und damit verbunden und charakterisiert durch eine erhöhte Zahl von Typ-I-K$^+$-Kanälen,

b) Behandlung der Zellkultur mit einem oder mehreren Testmaterialien einer Serie, die die Typ-I-K$^+$-Kanäle davon potentiell modulieren können,

c) Überwachen der Wirkung der Testmaterialien auf die Typ-I-K$^+$-Kanäle und

d) Auswahl von chemischen Stoffen von der Serie von Testmaterialien, die die Typ-I-K$^+$-Kanäle modulieren können.

**2.** Assay nach Anspruch 1, dadurch gekennzeichnet, daß die Überwachung der Stufe c) durch Messung des elektrischen Stroms durch die K$^+$-Kanäle, die mit dem Testmaterial befeuchtet sind, erfolgt.

**3.** Assay nach Anspruch 2, dadurch gekennzeichnet, daß die Auswahl der Stufe d) auf Testmaterial, das wenig oder weniger als normal elektrischen Strom durch die Typ-I-K$^+$-Kanäle induziert, oder auf der Änderung der Intensität eines Membranpotential-Erkennungsfarbstoffs oder eines Calciumerkennungsfarbstoffs beruht.

**4.** Assaysystem zur Bestimmung des Vorhandenseins eines Zustands einer Autoimmunkrankheit in einem Individuum, umfassend die Stufen:

a) Bereitstellung von T-Zellen, die K$^+$-Ionenkanäne enthalten, von einem Testindividuum,

b) Identifizierung abnormaler doppelt-negativer T-Zellen, die einen Überschuß an Typ-I-K$^+$-Kanälen zeigen, von der Population der T-Zellen der Stufe a),

c) Messung der relativen Zahlen der Typ-I-K$^+$-Kanäle der in Stufe b) identifizierten Zellen und

d) Bestimmung, ob die Typ-I-K$^+$-Xanäle, verglichen mit normal, erhöht sind.

**5.** Assay nach Anspruch 4, dadurch gekennzeichnet, daß die Messung der Stufe c) durch Messung des elektrischen Stroms durch die Typ-I-K$^+$-Kanäle erfolgt.

**6.** Assay nach Anspruch 5, dadurch gekennzeichnet, daß die Bestimmung der Stufe d) auf der Messung des elektrischen Stroms durch Typ-I-K$^+$-Kanäle oder auf der Intensität der Farbstofffärbung in einer normalen, phänotypisch ähnlichen T-Zelle erfolgt.

**7.** Verwendung eines extrinsischen Materials für die Herstellung eines Arzneimittels, das für die Behandlung einer Autoimmunkrankheit nützlich ist, wobei das extrinsische Material eine Modulations-

wirkung auf Typ-I-K⁺-Kanäle in abnormalen doppelt-negativen T-Zellen zeigt, die einen Überschuß an Typ-I-K⁺-Kanälen zeigen und mit einer Autoimmunkrankheit verbunden sind.

**8.** Verwendung nach Anspruch 7, dadurch gekennzeichnet, daß das Material mit einem Cytotoxin konjugiert ist und einen abnormalen doppelt-negativen, T-Zellen-Typ-I-spezifischen Antikörper für die Lokalisierung der abnormalen doppelt-negativen T-Zellen aufweist.

**9.** Verwendung nach Anspruch 7, dadurch gekennzeichnet, daß das Material Tetraethylammonium ist.

**10.** Verwendung nach Anspruch 7, dadurch gekennzeichnet, daß die Autoimmunkrankheit systemischer Lupus erythematodes ist.

**11.** Verwendung nach Anspruch 7, dadurch gekennzeichnet, daß die Autoimmunkrankheit Typ-I-Diabetes-mellitus ist.

**12.** Verwendung nach Anspruch 7, dadurch gekennzeichnet, daß die Autoimmunkrankheit kollagenrheumatoide Arthritis ist.

**13.** Verwendung nach Anspruch 7, dadurch gekennzeichnet, daß die Autoimmunkrankheit Multiple sklerose (EAE-Enzephalomyelitis) ist.

**14.** Verwendung nach Anspruch 7, dadurch gekennzeichnet, daß die Autoimmunkrankheit Myasthenia gravis ist.

**15.** Verwendung nach Anspruch 7, dadurch gekennzeichnet, daß das Material ein chemischer Stoff ist, der entsprechend dem Assay nach Anspruch 1 identifiziert wurde.

**16.** Verfahren zur Verarbeitung eines chemischen Stoffes, der entsprechend dem Assay nach Anspruch 1 ausgewählt wurde, bei der Herstellung einer Zusammensetzung, die den chemischen Stoff als wesentliche Komponente enthält, wobei die Zusammensetzung nützlich ist, wenn sie in vivo mit abnormalen doppelt-negativen T-Zellen, die einen Überschuß an Typ-I-K⁺-Kanälen enthalten, des Immunsystems in Kontakt kommt, ihre Modulationseigenschaften zu vermitteln.

**17.** Verwendung einer Zusammensetzung erhalten nach dem Verfahren nach Anspruch 16 für die Herstellung eines Arzneimittels zur Behandlung einer Autoimmunkrankheit.

**Revendications**

**1.** Test de criblage et d'identification de matériaux extrinsèques ayant un effet modulateur sur les canaux d'ions K⁺ de type *I* des cellules T doublement négatives (CD₄CD₈) anormales, présentant un excès de canaux d'ions K⁺ de type *I*, lié à une maladie auto-immune, composé des étapes comprenant:

a) la préparation d'une culture de cellules T doublement négatives anormales, anormales du fait qu'elles présentent une caractéristique unique d'altération de canaux d'ions K⁺, et liées à une maladie auto-immune caractérisée par des nombres élevés de canaux d'ions K⁺ de type *I*,
b) la mise en contact de ladite culture de cellules avec une ou plusieurs des matériaux-tests d'une batterie, et qui peuvent moduler potentiellement les canaux d'ions K⁺ de type *I*, de celles-ci,
c) le monitoring de l'effet desdits matériaux-tests sur lesdits canaux d'ions K⁺ de type *I*, et
d) la sélection des candidats au sein de la batterie de matériaux-tests, capables de moduler les canaux d'ions K⁺ de type *I*.

**2.** Test selon la revendication 1, dans lequel le monitoring à l'étape c) est conduit en mesurant le courant électrique à travers les canaux d'ions K⁺ baignés par ledit matériau-test.

**3.** Test selon la revendication 2, dans lequel la sélection à l'étape d) est basée sur un matériau-test induisant peu de courant électrique ou moins de courant électrique que la normale, à travers les canaux d'ions K⁺ de type *I*, ou sur le changement d'intensité d'un colorant sensible à un potentiel de membrane, ou d'un colorant sensible au calcium.

**4.** Test de détermination de la présence d'un état de maladie auto-immune chez un individu, composé des étapes comprenant:

a) la préparation de cellules T contenant des canaux d'ions K⁺, à partir d'un individu test,
b) l'identification des cellules T doublement négatives anormales présentant un excès de canaux d'ions K⁺ de type *I*, au sein de la population de cellules T de l'étape a),
c) la mesure des nombres relatifs de canaux d'ions K⁺ de type *I* des cellules identifiées à l'étape b), et
d) la détermination du fait que lesdits canaux d'ions K⁺ de type *I* sont en nombre élevé par rapport à la normale.

**5.** Test selon la revendication 4, dans lequel la mesure à l'étape c) est conduite par des mesures de courant électrique à travers les canaux d'ions K$^+$ de type *I*.

**6.** Test selon la revendication 5, dans lequel la détermination à l'étape d) est basée sur une mesure de courant électrique à travers les canaux d'ions K$^+$ de type 1, ou sur l'intensité de la coloration au pigment dans une cellule T normale, phénotypiquement similaire.

**7.** Utilisation d'un matériau extrinsèque pour la préparation d'un médicament utile pour le traitement de maladie auto-immune, ledit matériau extrinsèque ayant un effet modulateur sur le canal d'ions K$^+$ de type *I* dans les cellules T doublement négatives anormales, présentant un excès de canaux d'ions K$^+$ de type *I*, lié à une maladie auto-immune.

**8.** Utilisation selon la revendication 7, dans laquelle ledit matériau est conjugué à une cytotoxine et possède un anticorps spécifique des cellules T de type *I* doublement négatives, anormales, pour détecter lesdites cellules T doublement négatives, anormales.

**9.** Utilisation selon la revendication 7, dans laquelle ledit matériau est le tétraéthylammonium.

**10.** Utilisation selon la revendication 7, dans laquelle ladite maladie auto-immune est le lupus érythémateux systémique.

**11.** Utilisation selon la revendication 7, dans laquelle ladite maladie auto-immune est le diabète sucré de type 1.

**12.** Utilisation selon la revendication 7, dans laquelle ladite maladie auto-immune est l'arthrite rhumatoïde collagène.

**13.** Utilisation selon la revendication 7, dans laquelle ladite maladie auto-immune est la sclérose multiple (encéphalomyélite EAE).

**14.** Utilisation selon la revendication 7, dans laquelle ladite maladie auto-immune est la myasthénie grave.

**15.** Utilisation selon la revendication 7, dans laquelle ledit matériau est un candidat identifié conformément au test défini dans la revendication 1.

**16.** Procédé comprenant l'incorporation d'un candidat choisi selon le test défini dans la revendication 1, lors de la préparation d'une composition contenant ledit candidat en tant que composant essentiel, ladite composition étant utile pour conférer ses propriétés modulatrices, lors de sa mise en contact in vivo avec des cellules T doublement négatives, anormales, d'un système immunitaire, contenant un excès de canaux d'ions K$^+$ de type *I*.

**17.** Utilisation d'une composition obtenue par le procédé tel que défini dans la revendication 16, pour la préparation d'un médicament pour le traitement d'une maladie auto-immune.

FIG. 1

FIG. 2

$$CD4^-CD8^-Thy-1.2^+$$

*FIG. 3*

FIG. 4

_FIG. 5_

EP 0 463 114 B1

CD4⁻CD8⁻Thy−1.2⁺

*FIG. 6*

EP 0 463 114 B1